# EUROPEAN PATENT APPLICATION

(11) **EP 4 316 446 A1**
(43) Date of publication of application: **07.02.2024**
(21) Application number: 23186945.4
(22) Date of filing: 21.07.2023
(51) Int. Cl.: A61G 15/14

(54) **DENTAL TREATMENT UNIT PROVIDED WITH AN NFC DEVICE**

(30) Priority: 03.08.2022 IT 202200016206
(71) Applicant: CEFLA SOCIETA' COOPERATIVA, 40026 Imola (BO) (IT)
(72) Inventor: COLAZZO, Giorgio, 40026 Imola (BO) (IT); SCIARRA, Francesco, 40026 Imola (BO) (IT)
(74) Representative: Del Nero, Susanna

(57) **Abstract**

Dental treatment unit (10) comprising:
- a dentist's board (4) preferably provided with a graphical interface (45) for interaction with a human operator,
- optionally a scialytic lamp (12),
- a hydrogroup (2),
- a plurality of electronic boards connected through a BUS,
- a patient chair (1), in its turn comprising a seat (7) that can be raised/lowered with respect to the ground, a backrest (8), and an arch arm (9) allowing said backrest to assume a position substantially parallel to the ground, a position substantially perpendicular to the ground, and all the intermediate positions between these two extreme positions,
**characterized by**
being provided with an NFC reader configured to automatically activate the communication with at least one NFC tag carried by a human operator, when said NFC tag is approached to said NFC reader at a reading range shorter than 10 cm, said NFC reader being configured for registering said human operator carrying said NFC tag at a first connection with said dental unit (10).

## Description

The present invention relates to the technical field of dental treatment units, and in particular to a dental treatment unit provided with an NFC device, allowing the recognition of dental operators by said dental treatment unit.

Dental treatment units have been industrially produced for several decades, and comprise a plurality of instruments allowing a dentist and/or a dental hygienist to administer dental therapies to a patient. Typically, they comprise a patient chair, a hydrogroup, a scialytic lamp, a dentist's board, an assistant's board generally provided with suction cannulas.

Said patient chair typically comprises a seat and a backrest which moves with respect to said seat, so that said backrest can be brought in a substantially perpendicular position with respect to floor, in a substantially parallel position with respect to floor, and in all intermediate positions between these two extreme positions. Said movements typically occur through an arch arm connecting said seat to said backrest. Said seat can be raised/lowered with respect to floor.

Said hydrogroup is a structural portion of dental treatment units, which generally allows to support the dental treatment unit portions different from patient chair, i.e. an assistant's board, a scialytic lamp, optionally a dentist's board, which in its turn supports a plurality of dental handpieces (dental syringe, micro-motor, turbine, polymerizing lamp, dental camera, etc.). Often the hydrogroup also supports a bowl and a water-to-cup spout. Said hydrogroup is substantially a hollow structure housing reservoirs, electronic boards, piping and wiring. Said components are hidden from view, so that the dental treatment unit has a pleasing and reassuring look for patients.

In the art, it is known that dental treatment units are provided with a graphical interface for human operators, preferably placed on the dentist's board, provided for giving commands to the unit and also for showing messages concerning the settings and the working of the unit itself to dental operators.

In the art, it is known that dental treatment units are provided with hygiene systems for the water provided by the dentist's instruments placed on the dentist's board. It is further known that the water provided by said instruments is warmed up in order to bring it from room temperature to a temperature nearer to body temperature (from about 20°C to about 37°C).

In the art, it is known that dental treatment units are provided with hygiene programs aimed at disinfecting the piping channelling water from the connection box to the instruments placed on the dentist's board and on the assistant's board. Normally, such programs are activated when the dental treatment unit is not accommodating a patient. Hygiene programs comprise a pre-set sequence of steps: emptying pipes from water - refilling them with disinfectant - contact between disinfectant and piping for a pre-set time - emptying from disinfectant - rinsing. Normally such hygiene programs are activated by a dental assistant during the resetting of the dental treatment unit after treating a patient.

In the art, dental treatment units are known comprising a plurality of electronic boards intended for controlling the sundry portions of the dental treatment unit. On said electronic boards there are provided microcontrollers and microprocessors; said electronic boards are connected through a BUS, e.g. a CAN-BUS or a LIN-BUS.

It is worth noting that, generally in the morning, all the dental treatment units installed in a dental practice are switched on, each through its own main switch. At that point, generally the switched on dental treatment units enter in an energy-saving stand-by mode, wherein e.g. the graphical interface placed on the dentist's board is switched off and cannot be used; the scialytic lamp is switched off; hygiene systems are deactivated; the water provided to dentist's instrument is not warmed up.

In the art, it is known that dental practices are provided with a management software managing personal details, clinical records and images of each patient. Such data can be viewed on a dedicated PC, but also on the graphical interface placed on the dentist's board of the dental treatment unit or on a screen optionally provided on said dental treatment unit.

In the art, it is known that dental treatment units are provided with sundry optional features that can be installed on the dental treatment unit itself at the time of purchase: alternatively, optional features can be added/activated in a second moment, following the installation of the dental treatment unit in the dental practice. In this case, normally a hardware key is requested in order to activate the new functions on the dental treatment unit.

In the art, dental treatment units provided with voice control are known. Some voice commands, if given in a wrong moment, can affect patient's safety: e.g. moving patient's chair while the dentist is holding an active rotating instrument inside the patient's oral cavity. For this reason, voice commands normally require a consent by the dental operator who is treating the patient at that very moment, e.g. the foot control must be touched by the dental operator.

In the world, there are provided even very wide dental practices, wherein a plurality of dentists and dental hygienists cooperate. Often professionals have different dental specialties, like restorative dentistry, endodontics, orthodontics, dental prosthetics, etc. Often in a dental practice there is provided a co-presence of people hired from the practice and professionals working in the dental practice as freelance. Each professional uses one or more of the dental treatment units installed in the dental practice while providing dental therapies to patients.

Every human dental operator working in a given dental practice has her/his own preferences concerning the setting of the dental treatment unit, e.g. a given height of the seat of the dental patient chair, a given degree of reclining of the backrest, given working parameters of dental instruments placed on dentist's board, automatic settings of scialytic lamp, bowl, water glass, foot control, etc.

In the art, it is known performing maintenance interventions, which can be performed in a preventative way, after a pre-set number of working hours, or on call because the dental treatment unit stopped working or is malfunctioning. Generally, a service technician travels to the dental practice and performs her/his intervention. The maintenance intervention might require the download of firmware/software files, or new firmware/software files might become available, e.g. in order to correct bugs in said firmware/software.

It is worth mentioning that in wide dental practices, often dental treatment units are placed one beside the other, inside operating spaces ranging 9 to 16 m², therefore with dimension ranging about 3x3 m a 4x4 m. Often dental treatment units are placed near to one another, with distances between units that can be shorter than 2 m.

WO2005096177A1 of Planmeca discloses a dental treatment unit provided with an RFID reader and RFID tags worn by dental operators.

WO201361887A1 of Morita discloses a dental treatment unit provided with a multi-controller comprising a plurality of dental devices; a memory unit containing information on dental operators and patients and information on said dental devices, and a control unity that, according to the quoted information, shows a screen provided on the dental device so as to reflect a setting made by a dental operator.

US2018357384 of KaVo Dental Technologies describes a portable universal controller, including a memory having a graphical user interface generator and control software for a plurality of dental devices, including a dental treatment unit, dental hand-pieces provided as stand-alone units, X-ray sensors, 2-D imaging devices, 3-D panoramic imaging devices, etc. Said controller can comprise an NFC connection.

US2010070903 of Planmeca describes a dental unit comprising a microprocessor arranged to control the dental unit according to a user-specific profile, and the dental unit comprises a function for reading the user profile from an external record, which can be an USB memory stick or a record readable via a data network.

Aim of the present invention is providing a dental treatment unit allowing said unit to automatically recognize the single human operator among the staff of the dental practice.

This object is achieved by an apparatus and a method having the features of the independent claims. Advantageous embodiment and refinements are specified in the claims dependent thereon.

The aim is solved by providing an NFC device comprising an NFC reader placed on a dental treatment unit, and at least an NFC tag, preferably a wearable device worn by human operators, capable of authenticating a human operator both on the dental treatment unit and on management programs when suitably interfaced. Human operators wear an NFC tag that, when placed near said NFC reader, recognizes the human operator interacting at that moment with the dental treatment unit, and allows a rapid access to a series of menus. E.g., the dental treatment unit performs the uploading of the configuration of the dental treatment unit associated with a given user and/or can authenticate the same operator on the management software. This accelerates the operations of authentication and operator set-up, especially in a dental practice wherein a plurality of dental operators works.

In this case, NFC technology was preferred to RFID, in that NFC technology has a short-range working (shorter than 10 cm) and operates at about 13.56 MHz, and therefore is subject to less interferences. Moreover, NFC technologies has the possibility of using a cryptographic infrastructure that allows and ensures also bi-directional transfers (both read and write, communications peer to peer) and is more suitable for transferring data in an application like the one described. With respect to other file transfer protocols, like e.g. Bluetooth, NFC technology is less energy-consuming.

NFC technologies available on the market comprise:
- NXP's Mifare operates at the 13.56 MHz frequency, and is built on the ISO 14443 Type A standard;
- HID's iCLASS platform operates at the 13.56 MHz frequency, but it uses the less common ISO 15693 standard;
- LEGIC's platform operates at the 13.56 MHz frequency, and is compliant with ISO 14443 and ISO 15693 standards;
- Sony's FeliCa operates at the 13.56 MHz frequency and is based on the ISO 18902 standard;
- Open systems based on ISO 14443 standard.

In the preferred embodiment, the standard Mifare 1K Classic was used.

The working is ensured by the presence of an NFC tag that is read by said NFC reader placed in the receiver electronic board housed in the dental treatment unit, which in its turn is connected to the BUS allowing the communication with the dental treatment unit.

Each NFC tag has its own unique identifier that can be associated to a specific human operator. For an effective use, the NFC tag must be approached to the NFC reader at a distance shorter than 10 cm, preferably 5 cm.

In its preferred embodiment, said NFC tag is provided in the form of a device wearable by human operators, preferably in the form of a bracelet. In a further embodiment, the NFC tag can be provided in any wearable device, like a smart watch. Alternatively, the NFC can be provided in the form of a badge to be carried in the pocket of the white coat of the human operator, or can be embedded in a smart phone.

In a preferred embodiment, the NFC reader is placed inside the dentist's board. Nonetheless, alternative placements are possible, e.g. in the top portion of the hydrogroup, or in the assistant's table, or anyway in a position that is about at the height of the hands of a human operator.

The advantages of the present invention are manifold.

A first advantage is linked to the sensitivity to disturbances and to the distances at play. In fact, NFC technology has a working range that is normally shorter than 10 cm, and this make it insensitive to disturbances coming from longer distances.

A second advantage is that with NFC technology, communication is bidirectional, i.e. goes from the tag to the medical device, but also from the medical device to the tag. This allows the transfer e.g. of software files.

A third advantage is the possibility of interrupting the stand-by mode or the hygiene mode of the dental treatment unit by approaching the NFC tag of a human operator to the NFC reader placed on the unit itself.

The fourth advantage is the possibility of associating a set of parameters for the set-up of the different instruments provided on the dental treatment unit to a given dental operator.

The fifth advantage is the reduction of interaction times between dental operator and menu of the dental treatment unit, in that shortcuts can be activated for setting the different instruments, e.g. the foot control mode.

The sixth advantage is the possibility for dental staff of logging on in the management software of the dental practice through the NFC tag, therefore visualizing the folders containing the data of each patient.

The seventh advantage is, when a dental treatment unit is provided with voice control, the possibility of giving consent to a voice command by using the NFC tag, which is approached to the NFC reader placed on the dentist's board or in another configuration as above described.

The eighth advantage is activating through NFC tag said optional functionalities, operation which up to now used to require the use of a hardware key.

The ninth advantage is the possibility of performing maintenance interventions on the dental treatment unit in a mixed mode: the service technician on site performs an access through her/his NFC tag containing specific authorizations, while a remote colleague can act on the firmware/software installed in the same dental treatment unit.

Further advantages and properties of the present invention are disclosed in the following description, in which exemplary embodiments of the present invention are explained in detail based on the drawings:
- Figure 1: Lateral view of a typical dental treatment unit;
- Figure 2: NFC symbol placed in proximity of the NFC reader;
- Figure 3: Example of a layout of a dental practice provided with a plurality of dental treatment units;
- Figure 4: Examples of menus that can be viewed on the graphical interface;
- Figure 5: Example of interaction between human operator and dental treatment unit.

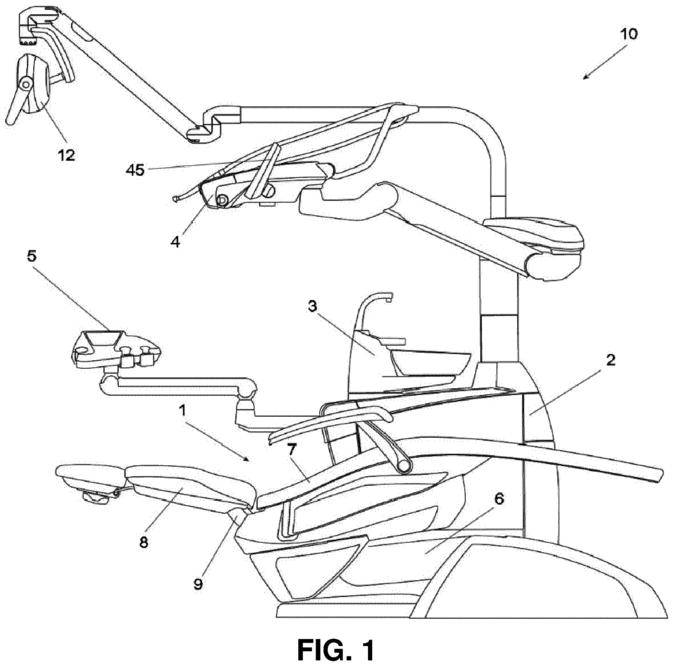

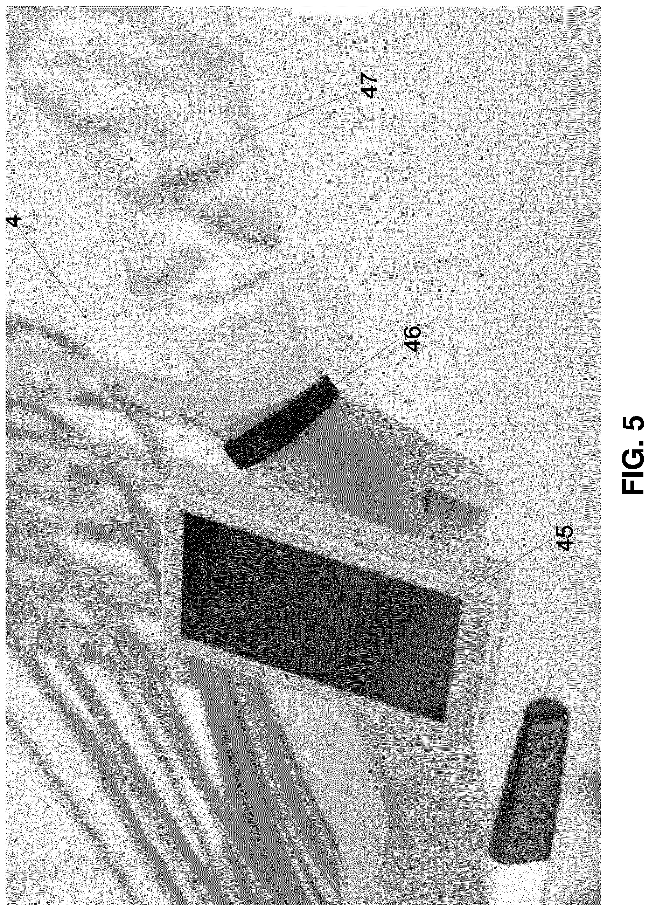

Figure 1 shows a dental treatment unit 10, in particular a chair mounted dental treatment unit. Said chair mounted dental treatment unit comprises: a patient chair 1, a hydrogroup 2, a bowl 3, a dentist's board 4 preferably provided with a graphical interface 45 (more visible in Figure 5), an assistant's board 5, and a scialytic lamp 12. Patient's chair seat 7 and backrest 8 move with respect to each other through an arch arm 9. Optionally said dental unit further comprises a screen, on which patient's data can be visualized; such data are memorized in a management software of the dental practice.

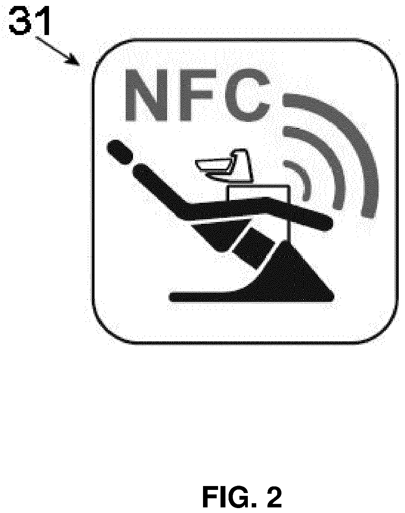

In the dentist's board 4 there is provided an NFC reader allowing the interaction with at least an NFC tag carried by a human operator. In an embodiment, said NFC reader is placed on the side of the dentist's board 4, while in another embodiment the NFC reader is placed on the rear of the dentist's board 4. On the dentist's board 4 there is provided a symbol indicating to human operators the position whereto said NFC tag must be approached. Said NFC symbol 31, which measures about 20x20 mm, is shown in Figure 2.

On the dental treatment unit 10 there is provided an electronic board containing an NFC reader. Said electronic board is connected to the BUS of the dental treatment unit. In the preferred embodiment, said electronic board comprising the NFC reader is placed inside the dentist's board 4. Given the low range of sensitivity, the dental operator can easily approach her/his tag to the NFC reader. Nonetheless, different placements are possible, e.g. in the top portion of the hydrogroup 2 or in the assistant's board 5, anyway in a position that is about at the height of the dental operator's hands.

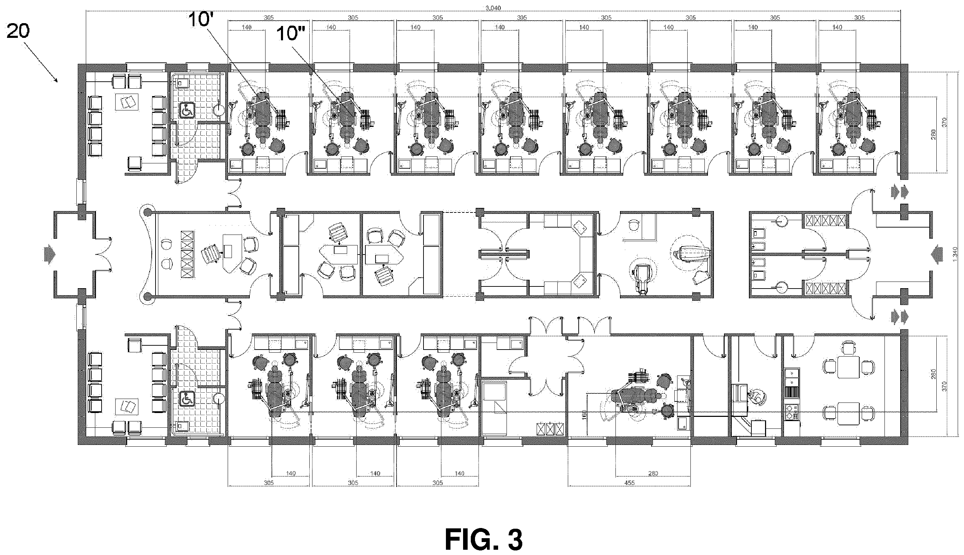

Figure 3 shows an example of a layout 20 of a dental practice provided with a plurality of dental treatment units 10, in this particular case provided with twelve dental treatment units 10. From the layout, which is just an example, is easy to gather that the distance between two units 10 is shorter than 2 m.

By observing the layout of Figure 3 is easy to understand why a short transmission range is so crucial, in that when two human operators operate on two adjacent dental treatment units 10' and 10", there is the danger that the first operator, who is working on the unit 10', gives a command that might affect the second unit 10" on which the second dental operator is working, endangering the patient that is laying on the second unit 10". The short sensitivity range, lower than 10 cm, prevents this danger.

At the first use of a dental treatment unit 10' by a new operator, e.g. Dr Bianchi, Dr Bianchi approaches his NFC tag to the icon 31 placed on the dentist's board 4.

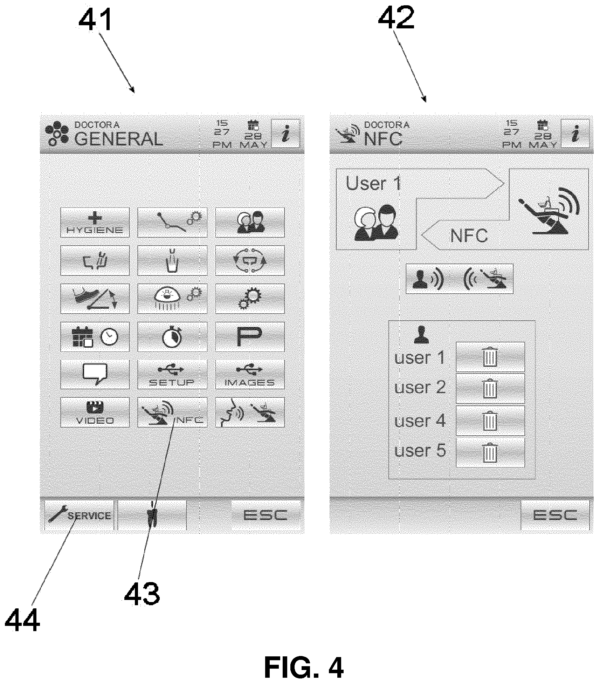

This activates a menu like the one shown in Figure 4, that first allows to type through a (not shown) keyboard available on the graphical interface the name of Dr Bianchi, and then to choose a series of preferred settings for each of the instruments provided on the unit 10 (e.g. curing lamp and/or scialytic lamp intensity, number of turns of micro-motor/turbine, height and degree of reclining of the patient chair seat, etc.).

The univocal association between tag and human operator, e.g. Dr Bianchi, occurs the first time when a given human operator approaches the dental treatment unit 10': the operator must manually insert her/his name on said dental treatment unit 10', and this establishes a biunivocal connection between operator's tag and unit 10'. When Dr Bianchi wishes to operate on a second unit 10", the same operation must be repeated. In other words, the tag is anonymous per se: only in connection with a given dental treatment unit 10' or 10" the NFC tag is recognized as belonging to a given operator.

Figure 4 shows two examples of menus 41, 42, that can be visualized on the graphical interface 45 of the dentist's board 4.

The first menu 41 comprises an icon 43; if clicked, it leads to the second menu 42, allowing, as explained above, the biunivocal connection between a given dental treatment unit 10', 10" and a given operator (User 1) who can manually type her/his name and surname.

In case of a maintenance intervention, a service technician goes to the dental practice and logs in through her/his NFC tag, opening a maintenance menu normally not accessible by the staff in the dental practice. Now remote interventions become possible, e.g. downloading, e.g. from a cloud, a series of files, like e.g. firmware/software updates, or uploading the error log on the cloud, or downloading the hygiene cycles performed by the dental treatment unit.

An icon 44 allows said service technician to activate the Service menu, so as to perform a diagnostic of the dental treatment unit, or to perform a download of an error log, or to perform an upload of new firmware/software files.

Figure 5 shows an example of interaction between a human operator 47 and a dental treatment unit 10 provided with a dentist's board 4. As explained above, said dentist's board 4 preferably comprises a graphical interface 45 for issuing commands to a dental treatment unit 10 and for visualizing the settings of said dental treatment unit 10. In this Figure there is shown the case wherein the NFC tag is inside a bracelet 46 worn on a wrist of a human operator 47. Although the NFC tag can also be a badge carried in the pocket of the white coat of the operator, the bracelet or anyway a wearable device is the best solution from the hygienic point of view, in that it does not need to be touched by operator's hands, which could be contaminated by patient's blood/saliva.

The method according to the present invention comprises the following steps:
- Providing a dental treatment unit 10 provided with an NFC reader connected to the BUS of said dental treatment unit;
- Providing at least an NFC tag that can be read by said NFC reader;
- Bringing said NFC tag in proximity of the NFC reader on the unit 10, so as to establish a contact;
- At the first connection, performing the registration of said NFC reader on said dental treatment 10 by inserting the name of the dental operator;
- The contact between NFC reader and NFC tag worn by a human operator allows to perform the following operations:
   ∘ Stopping said stand-by or hygiene mode; and/or
   ∘ Setting a series of working parameters of the different instruments provided on said dental treatment unit, e.g. intensity of the curing lamp and/or scialytic lamp, number of turns of micro-motor/turbine, height of the dental chair seat, degree of reclining of the backrest, etc., even using shortcuts; and/or
   ∘ Connecting to the management software of the dental practice, containing data and images of patients; and/or
   ∘ When said dental treatment unit is provided with a module for voice commands, giving consent to the voice command by approaching said NFC; and/or
   ∘ Activating, through said NFC tag, optional modules/functionalities on said dental treatment unit 10, at the same time or successively to the installation of said unit 10 in the dental practice; and/or
   ∘ In case of a maintenance intervention by a service technician, activating, through the contact between NFC reader and tag, the possibility for the technician to act on the firmware/software of the dental treatment unit 10, performing file download and upload.

In an alternative embodiment, each patient is provided with an NFC tag. When the patient takes place in the patient's chair 1 of a dental treatment unit, the approaching between NFC tag and reader triggers:
- the stop of the stand-by mode of the dental treatment unit 10 (the scialytic lamp 12 is switched on, the graphical interface on the dentist's board 4 is activated and can be used to visualize information/issue commands);
- when said dental treatment unit 10 is provided with voice control, the emission of a voice message welcoming the patient;
- the visualization of the clinical record of the patient seated on the dental patient chair on the same graphical interface 45 or on a (not shown) optional screen provided on the dental treatment unit 10.

With reference to the above-described embodiments, in combination with any of them, the NFC tag associated with dental staff, service technicians and/or patients can be in the form of the NFC system embedded in a cell phone like a smartphone or similar. Generally, cell phones are natively provided with an NFC system and also contain the identifying data of the owner.

In an embodiment, said cellular phone can be provided with an APP for interfacing with a dental treatment unit and/or with the dental practice management software, and can even work as communication centre among user, dental treatment unit and management centre working under the direct control and supervision of the user.
- 1: chair
- 2: hydrogroup
- 3: bowl
- 4: dentist's board
- 5: assistant's board
- 6: pantograph
- 7: seat
- 8: back rest
- 9: arch arm
- 10: dental treatment unit
- 12: scialytic lamp
- 20: dental practice layout
- 31: NFC reader symbol
- 41: first menu
- 42: second menu
- 43: icon
- 44: icon
- 45: graphical interface
- 46: bracelet with NFC tag
- 47: human operator

## Claims

1. Dental treatment unit (10) comprising:
- a dentist's board (4) preferably provided with a graphical interface (45) for interaction with a human operator,
- optionally a scialytic lamp (12),
- a hydrogroup (2),
- a plurality of electronic boards connected through a BUS,
- a patient chair (1), in its turn comprising a seat (7) that can be raised/lowered with respect to the ground, a backrest (8), and an arch arm (9) allowing said backrest to assume a position substantially parallel to the ground, a position substantially perpendicular to the ground, and all the intermediate positions between these two extreme positions,
**characterized by**
being provided with an NFC reader configured to automatically activate the communication with at least one NFC tag carried by said human operator when said NFC tag is approached to said NFC reader at a reading range shorter than 10 cm, said NFC reader being configured for registering said human operator carrying said NFC tag at a first connection with said dental unit (10).

2. Dental treatment unit (10) according to claim 1, wherein the NFC technology is chosen from the group consisting of NXP's Mifare, HID's iCLASS, LEGIC's platform, Sony's FeliCa, Open systems.

3. Dental treatment unit (10) according to claim 1, wherein said NFC tag carried by said human operator is housed in a bracelet (46) worn by said human operator (47), or in another wearable device like a smart watch, or alternatively is in the form of a badge carried in a pocket of said human operator or is embedded in a cell phone or smart watch provided with a specific APP.

4. Dental treatment unit (10) according to one or more of claims 1-3, wherein said NFC reader is housed inside said dentist's board (4).

5. Dental treatment unit (10) according to one or more of claims 1-3, wherein said NFC reader is housed inside an assistant's board (5) or inside said hydrogroup (2).

6. Method for the use of the dental treatment unit (10) according to one or more of claims 1-5, comprising the following steps:
- Providing said dental treatment unit (10) provided with said NFC reader connected to the BUS of said dental treatment unit;
- Providing at least a said NFC tag that can be read by said NFC reader;
- Bringing said NFC tag in proximity of the NFC reader on the unit (10), so as to establish a contact;
- At the first connection, performing the registration of said NFC reader on said dental treatment (10) by inserting the name of the dental operator carrying said NFC tag;
- The contact between NFC reader and NFC tag worn by said human operator allowing to perform one or more of the following operations:
∘ Stopping a stand-by or hygiene mode of said dental treatment unit (10); and/or
∘ Setting a series of working parameters of the different instruments provided on said dental treatment unit, and/or
∘ Connecting to a management software of the dental practice, containing data and images of patients; and/or
∘ When said dental treatment unit is provided with a module for voice commands, giving consent to the voice command by approaching said NFC; and/or
∘ Activating, through said NFC tag, optional modules/functionalities on said dental treatment unit (10).

7. Method for the use of the dental treatment unit (10) according to claim 6, wherein said NFC tag is carried by a service technician: , through the contact between NFC reader and tag, said technician is allowed to act on the firmware/software of said dental treatment unit (10), downloading and uploading files.

8. Method for the use of the dental treatment unit (10) according to claim 6, wherein said NFC tag is carried is carried by a patient; when said patient takes place in said patient chair (1) of said dental treatment unit (10), the approaching of said NFC tag to said NFC reader triggers:
- the stop of the stand-by mode of the dental treatment unit (10);
- when said dental treatment unit 10 is provided with voice control, the emission of a voice message welcoming the patient;
- the visualization of the clinical record of the patient seated on the dental patient chair on the same graphical interface (45) or on an optional screen provided on the dental treatment unit (10).
